(19) Europäisches Patentamt
European Patent Office
Office européen des brevets

(11) Veröffentlichungsnummer: **0 122 449**
**B1**

# (12) EUROPÄISCHE PATENTSCHRIFT

(45) Veröffentlichungstag der Patentschrift: **03.06.87**

(51) Int. Cl.[4]: **C 07 C 127/22**, C 07 C 157/12, C 07 C 149/34, A 01 N 47/34

(21) Anmeldenummer: **84102655.2**

(22) Anmeldetag: **12.03.84**

(54) **Benzoylharnstoffe.**

(30) Priorität: **19.03.83 DE 3309987**
**15.11.83 DE 3341276**

(43) Veröffentlichungstag der Anmeldung:
**24.10.84 Patentblatt 84/43**

(45) Bekanntmachung des Hinweises auf die Patenterteilung:
**03.06.87 Patentblatt 87/23**

(84) Benannte Vertragsstaaten:
**AT BE CH DE FR GB IT LI NL SE**

(56) Entgegenhaltungen:
**EP - A - 0 057 888**
**EP - A - 0 074 074**

**Patent Abstracts of Japan, Band 5, Nr. 75, 19. Mai 1981**

**Die Akte enthält technische Angaben, die nach dem Eingang der Anmeldung eingereicht wurden und die nicht in dieser Patentschrift enthalten sind.**

(73) Patentinhaber: **BAYER AG, Konzernverwaltung RP Patentabteilung, D-5090 Leverkusen 1 Bayerwerk (DE)**

(72) Erfinder: **Sirrenberg, Wilhelm, Dr., Wuppertaler Strasse 21, D-4322 Sprockhövel (DE)**
Erfinder: **Klauke, Erich, Dr., Eichendorffweg 8, D-5068 Odenthal (DE)**
Erfinder: **Becker, Benedikt, Dr., Metzkausenerstrasse 14, D-4020 Mettmann (DE)**
Erfinder: **Krehan, Ingomar, Dr., Ludwig-Jahn-Strasse 54, D-5000 Köln 40 (DE)**
Erfinder: **Stendel, Wilhelm, Dr., In den Birken 55, D-5600 Wuppertal 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

**Beschreibung**

Die vorliegende Erfindung betrifft neue substituierte 1-Phenyl-3-benzoyl-(thio)harnstoffe, mehrere Verfahren zu ihrer Herstellung und ihre Verwendung als Schädlingsbekämpfungsmittel, insbesondere als Insektizide und Akarizide.

Es ist bereits bekannt, dass bestimmte Benzoylharnstoffe insektizide Eigenschaften besitzen (vgl. z.B. EP 0 057 888).

Es wurden die neuen substituierten 1-Phenyl-3-benzoyl-(thio)harnstoffe der Formel (I)

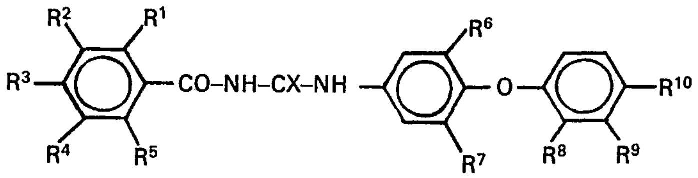

(I)

gefunden, in welcher

X für Schwefel oder Sauerstoff steht,

$R^1$ für Wasserstoff, Halogen, Nitro oder für einen Rest aus der Reihe $C_1$–$C_6$-Alkyl und $C_1$–$C_6$-Alkylthio steht,

$R^2$, $R^4$ und $R^5$ gleich oder verschieden sind und für Wasserstoff oder Halogen stehen,

$R^3$ für Wasserstoff, Halogen oder Nitro steht,

$R^6$ und $R^7$ gleich oder verschieden sind und für Wasserstoff, Halogen oder für einen $C_1$–$C_6$-Alkyl-Rest stehen,

$R^8$ und $R^9$ gleich oder verschieden sind und für Wasserstoff oder Halogen stehen und

$R^{10}$ für Chlordifluormethylthio, Trifluormethylthio, Chlordifluormethoxy oder Trifluormethoxy steht, wobei

(1) wenn X für Sauerstoff steht und

$R^2$, $R^3$, $R^4$, $R^6$, $R^8$ und $R^9$ für Wasserstoff stehen und

$R^7$ für Wasserstoff oder Halogen steht und

(a) $R^1$ und $R^5$ beide für Halogen stehen oder

(b) $R^1$ für Halogen, Nitro oder $C_1$–$C_6$-Alkyl und $R^5$ für Wasserstoff steht, in diesen Fällen den Rest

$R^{10}$ stets Chlordifluormethylthio oder Trifluormethylthio bedeutet und wobei

(2) wenn X für Sauerstoff oder Schwefel steht und

$R^6$ für Chlor steht und

$R^8$ für Wasserstoff oder Chlor steht und

$R^9$ für Wasserstoff steht und

$R^{10}$ für Chlordifluormethylthio, Trifluormethylthio, Chlordifluormethoxy oder Trifluormethoxy steht und

(a) $R^1$ und $R^3$ für Halogen und $R^2$, $R^4$ und $R^5$ für Wasserstoff stehen oder

(b) $R^5$ und $R^3$ für Halogen und $R^1$, $R^2$ und $R^4$ für Wasserstoff stehen oder

(c) $R^1$ und $R^4$ für Halogen und $R^2$, $R^3$ und $R^5$ für Wasserstoff stehen oder

(d) $R^2$ und $R^5$ für Halogen und $R^1$, $R^3$ und $R^4$ für Wasserstoff stehen, in diesen Fällen den Rest

$R^7$ stets Wasserstoff oder $C_1$–$C_6$-Alkyl bedeutet.

Diese neuen Verbindungen weisen starke biologische, insbesondere insektizide Eigenschaften auf, die ihre Verwendung als Schädlingsbekämpfungsmittel, insbesondere als Insektizide und Akarizide ermöglichen.

Weiterhin wurde gefunden, dass die neuen substituierten 1-Phenyl-3-benzoyl-(thio)harnstoffe der Formel (I) erhalten werden, indem man

a) substituierte Aniline der Formel (II)

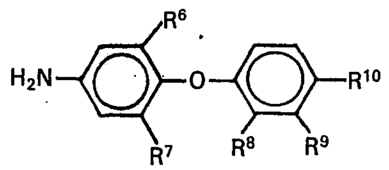

(II)

in welcher

$R^6$, $R^7$, $R^8$, $R^9$ und $R^{10}$ die oben angegebenen Bedeutungen haben, mit Benzoyl-iso(thio)cyanaten der Formel (III)

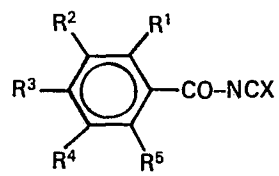

(III)

in welcher

X, $R^1$, $R^2$, $R^3$, $R^4$ und $R^5$ die oben angegebenen Bedeutungen haben, gegebenenfalls in Gegenwart eines Verdünnungsmittels umsetzt oder

b) substituierte Phenyl-iso(thio)cyanate der Formel (IV)

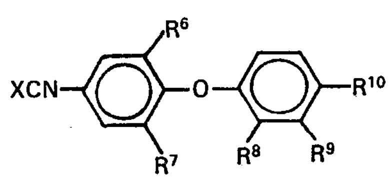

(IV)

in welcher

X, $R^6$, $R^7$, $R^8$, $R^9$ und $R^{10}$ die oben angegebenen Bedeutungen haben, mit Benzoesäureamiden der Formel (V)

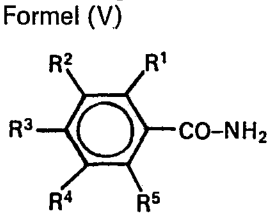

(V)

in welcher

$R^1$, $R^2$, $R^3$, $R^4$ und $R^5$ die oben angegebenen Bedeutungen haben, gegebenenfalls in Gegenwart eines Katalysators und gegebenenfalls in Gegenwart eines Verdünnungsmittels umsetzt.

Alkyl $R^1$, $R^6$ und $R^7$ und Alkylthio $R^1$ enthalten im Alkylteil geradkettiges oder verzweigtes Alkyl mit 1 bis 6, insbesondere 1 bis 4 Kohlenstoffatome. Beispielhaft seien Methyl, Ethyl, n-Propyl, i-Propyl, n-Butyl, i-Butyl, sec.-Butyl, tert.-Butyl, Methylthio, Ethylthio, n-Propylthio, i-Propylthio, n-Butylthio, i-Butylthio, sec.-Butylthio und tert.-Butylthio genannt.

Halogen bedeutet – wo nicht anders erläutert – Fluor, Chlor, Brom und Jod, vorzugsweise Fluor, Chlor und Brom.

Bevorzugt steht X für Sauerstoff.

Die neuen Verbindungen der Formel (I) verfügen über Eigenschaften, die ihre Verwendung als Schädlingsbekämpfungsmittel ermöglichen, insbesondere zeichnen sie sich durch eine hervorragende insektizide und akarizide Wirksamkeit aus.

Halogen steht jeweils für Fluor, Chlor, Brom und/oder Jod, vorzugsweise für Fluor, Chlor und/oder Brom.

Besonders bevorzugt sind die Verbindungen der Formel (I), in welcher

X für Sauerstoff oder Schwefel steht,

$R^1$ für Wasserstoff, Fluor, Chlor, Brom, Nitro, Methyl, Ethyl, n-Propyl, i-Propyl, n-Butyl, i-Butyl, sec.-Butyl, tert.-Butyl, Methylthio, Ethylthio, n-Propylthio, i-Propylthio, n-Butylthio, i-Butylthio, sec.-Butylthio oder tert.-Butylthio steht,

$R^2$, $R^4$ und $R^5$ gleich oder verschieden sind und für Wasserstoff, Fluor, Chlor oder Brom stehen,

$R^3$ für Wasserstoff, Fluor, Chlor, Brom, oder Nitro steht,

$R^6$ und $R^7$ gleich oder verschieden sind und für Wasserstoff, Fluor, Chlor, Brom, Methyl, Ethyl, n-Propyl, i-Propyl, n-Butyl, i-Butyl, sec.-Butyl oder tert.-Butyl stehen,

$R^8$ und $R^9$ gleich oder verschieden sind und für Wasserstoff, Fluor, Chlor oder Brom stehen und

$R^{10}$ für Trifluormethylthio oder Trifluormethoxy steht, wobei

(1) wenn X für Sauerstoff steht und

$R^2$, $R^3$, $R^4$, $R^6$, $R^8$ und $R^9$ für Wasserstoff stehen und

$R^7$ für Wasserstoff, Fluor, Chlor oder Brom steht und

(a) $R^1$ und $R^5$ beide für Fluor, Chlor und/oder Brom stehen oder

(b) $R^1$ für Fluor, Chlor, Brom, Nitro oder die obengenannten Alkylreste und $R^5$ für Wasserstoff steht, in diesen Fällen der Rest

$R^{10}$ stets Trifluormethylthio bedeutet und wobei

(2) wenn X für Sauerstoff oder Schwefel steht und

$R^6$ für Chlor steht und

$R^8$ für Wasserstoff oder Chlor steht und

$R^9$ für Wasserstoff steht und

$R^{10}$ für Trifluormethylthio oder Trifluormethoxy steht und

(a) $R^1$ und $R^3$ für Fluor, Chlor oder Brom und $R^2$, $R^4$ und $R^5$ für Wasserstoff stehen oder

(b) $R^5$ und $R^3$ für Fluor, Chlor oder Brom und $R^1$, $R^2$ und $R^4$ für Wasserstoff stehen oder

(c) $R^1$ und $R^4$ für Fluor, Chlor oder Brom und $R^2$, $R^3$ und $R^5$ für Wasserstoff stehen oder

(d) $R^2$ und $R^5$ für Fluor, Chlor oder Brom und $R^1$, $R^3$ und $R^4$ für Wasserstoff stehen, in diesen Fällen der Rest

$R^7$ stets Wasserstoff oder die obengenannten Alkylreste bedeutet.

Ganz besonders bevorzugt sind die Verbindungen der Formel (I), in welcher

X für Sauerstoff steht,

$R^1$ für Wasserstoff, Fluor, Chlor, Brom, Nitro, Methyl oder Methylthio steht,

$R^2$, $R^4$ und $R^5$ für Wasserstoff, Fluor, Chlor oder Brom stehen,

$R^3$ für Wasserstoff, Fluor, Chlor oder Nitro steht,

$R^6$ und $R^7$ gleich oder verschieden sind und für Wasserstoff, Chlor oder Methyl stehen,

$R^8$ und $R^9$ gleich oder verschieden sind und für Wasserstoff, Fluor oder Chlor stehen, und

$R^{10}$ für Trifluormethylthio oder Trifluormethoxy steht, wobei

(1) wenn

$R^2$, $R^3$, $R^4$, $R^6$, $R^8$ und $R^9$ für Wasserstoff stehen und

$R^7$ für Wasserstoff oder Chlor steht und

(a) $R^1$ und $R^5$ beide für Fluor, Chlor und/oder Brom stehen oder

(b) $R^1$ für Fluor, Chlor, Brom, Nitro oder Methyl und $R^5$ für Wasserstoff steht, in diesen Fällen der Rest

$R^{10}$ stets Trifluormethylthio bedeutet und wobei

(2) wenn

$R^6$ für Chlor steht und

$R^8$ für Wasserstoff oder Chlor steht und

$R^9$ für Wasserstoff steht und

$R^{10}$ für Trifluormethylthio oder Trifluormethoxy steht und

(a) $R^1$ und $R^3$ für Fluor, Chlor und/oder Brom und $R^2$, $R^4$ und $R^5$ für Wasserstoff stehen oder

(b) $R^5$ und $R^3$ für Fluor, Chlor und/oder Brom und $R^1$, $R^2$ und $R^4$ für Wasserstoff stehen oder

(c) $R^1$ und $R^4$ für Fluor, Chlor und/oder Brom und $R^2$, $R^3$ und $R^5$ für Wasserstoff stehen oder

(d) $R^2$ und $R^5$ für Fluor, Chlor und/oder Brom und $R^1$, $R^3$ und $R^4$ für Wasserstoff stehen, in diesen Fällen der Rest

$R^7$ stets Wasserstoff oder Methyl bedeutet.

Verwendet man nach Verfahrensvariante (a) 4-(2-Chlor-4-trifluormethoxy-phenoxy) -3-methyl-anilin und 2,6-Difluorbenzoylisocyanat als Ausgangsstoffe, so kann der Reaktionsablauf durch das folgende Formelschema wiedergegeben werden:

a) 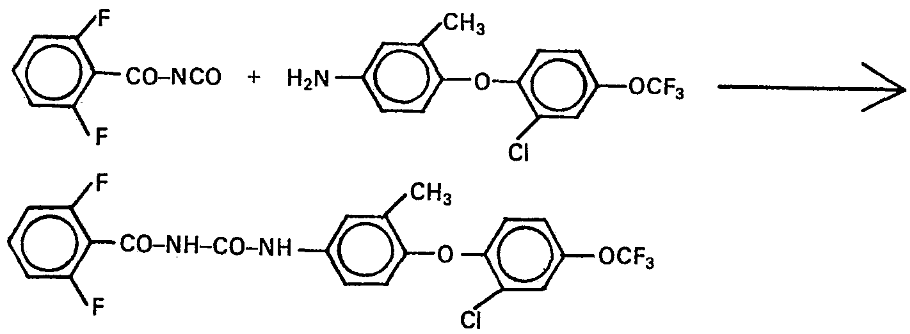

Verwendet man nach Verfahrensvariante (b) 4-(2-Chlor-4-trifluormethoxy-phenoxy) -3-methyl-phenylisothiocyanat und 2,6-Difluor-benzamid als Ausgangsstoffe, so kann der Reaktionsablauf durch das folgende Formelschema wiedergegeben werden:

b) 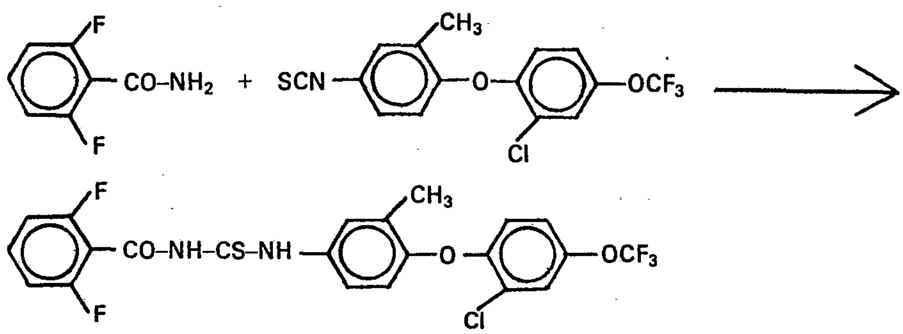

Als Beispiele für die Verbindungen der Formel (II) seien genannt:

Tabelle 1

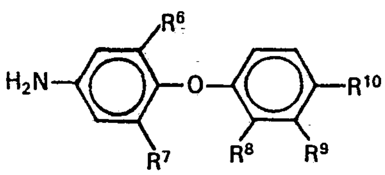

(II)

| $R^6$ | $R^7$ | $R^8$ | $R^9$ | $R^{10}$ |
|---|---|---|---|---|
| H | H | H | H | $OCF_3$ |
| Cl | H | H | H | $OCF_3$ |
| Cl | Cl | H | H | $OCF_3$ |
| H | Cl | Cl | H | $OCF_3$ |
| H | H | Cl | Cl | $OCF_3$ |
| Cl | H | H | Cl | $OCF_3$ |
| H | H | Cl | H | $OCF_3$ |
| H | H | H | Cl | $OCF_3$ |
| Cl | Cl | Cl | H | $OCF_3$ |
| Cl | Cl | H | Cl | $OCF_3$ |
| Cl | H | Cl | Cl | $OCF_3$ |
| Cl | Cl | Cl | Cl | $OCF_3$ |
| $CH_3$ | H | H | H | $OCF_3$ |
| $CH_3$ | Cl | H | H | $OCF_3$ |
| $CH_3$ | H | Cl | H | $OCF_3$ |
| $CH_3$ | H | H | Cl | $OCF_3$ |
| $CH_3$ | H | Cl | Cl | $OCF_3$ |
| $CH_3$ | Cl | Cl | H | $OCF_3$ |
| $CH_3$ | Cl | H | Cl | $OCF_3$ |
| $CH_3$ | Cl | Cl | Cl | $OCF_3$ |
| $CH_3$ | $CH_3$ | H | H | $OCF_3$ |
| $CH_3$ | $CH_3$ | Cl | H | $OCF_3$ |
| $CH_3$ | $CH_3$ | H | Cl | $OCF_3$ |
| $CH_3$ | $CH_3$ | Cl | Cl | $OCF_3$ |
| H | H | H | H | $SCF_3$ |
| Cl | H | H | H | $SCF_3$ |
| Cl | Cl | H | H | $SCF_3$ |
| H | Cl | Cl | H | $SCF_3$ |
| H | H | Cl | Cl | $SCF_3$ |
| Cl | H | H | Cl | $SCF_3$ |
| Cl | H | Cl | H | $SCF_3$ |
| H | H | Cl | H | $SCF_3$ |
| H | H | H | Cl | $SCF_3$ |
| Cl | Cl | Cl | H | $SCF_3$ |
| Cl | Cl | H | Cl | $SCF_3$ |
| Cl | H | Cl | Cl | $SCF_3$ |
| Cl | Cl | Cl | Cl | $SCF_3$ |
| $CH_3$ | H | H | H | $SCF_3$ |
| $CH_3$ | Cl | H | H | $SCF_3$ |
| $CH_3$ | H | Cl | H | $SCF_3$ |
| $CH_3$ | H | H | Cl | $SCF_3$ |
| $CH_3$ | H | Cl | Cl | $SCF_3$ |
| $CH_3$ | Cl | Cl | H | $SCF_3$ |
| $CH_3$ | Cl | H | Cl | $SCF_3$ |
| $CH_3$ | Cl | Cl | Cl | $SCF_3$ |
| $CH_3$ | $CH_3$ | H | H | $SCF_3$ |
| $CH_3$ | $CH_3$ | Cl | H | $SCF_3$ |
| $CH_3$ | $CH_3$ | H | Cl | $SCF_3$ |
| $CH_3$ | $CH_3$ | Cl | Cl | $SCF_3$ |

Die als Ausgangsstoffe zu verwendenden substituierten Aniline der Formel (II) sind bekannt und/oder lassen sich nach literaturbekannten Verfahren und Methoden herstellen (vgl. DE-OS 32 17 619, DE-OS 32 17 620, EP 00 57 588); die Aminogruppe kann nach üblichen Verfahren

in die Isocyanat- bzw. Iso-thio-cyanat-Gruppe umgewandelt werden, z.B. durch Umsetzung mit Phosgen bzw. Thiophosgen, wodurch die entsprechenden substituierten Phenyl-iso(thio)cyanate der Formel (IV) erhalten werden.

Als Beispiele für die Verbindungen der Formel (IV) seien die entsprechenden Isocyanate bzw. Iso-thio-cyanate der in Tabelle 1 aufgeführten substituierten Aniline der Formel (II) genannt.

Als Beispiele für die Verbindungen der Formel (III) seien genannt: 2-Fluor-, 2-Chlor-, 2-Brom-, 2-Nitro-, 2-Methyl-, 2-Methylthio-, 2,6-Difluor-, 2,6-Dichlor-, 2-Chlor-6-fluor-, 2-Chlor-4-nitro-, 3,4-Difluor-, 3,4-Dichlor-, 2,3,6-Trichlor-, 4-Fluor-, 4-Chlor-, 4-Brom- und 4-Nitrobenzoylisocyanat bzw. -benzoylisothiocyanat.

Die Ausgangsverbindungen der Formel (III) sind bekannt.

Als Beispiele für die Verbindungen der Formel (V) seien genannt: 2-Fluor-, 2-Chlor-, 2-Brom-, 2-Nitro-, 2-Methyl-, 2-Methylthio-, 2,6-Difluor-, 2,6-Dichlor-, 2-Chlor-6-fluor-, 2-Chlor-4-nitro-, 3,4-Difluor-, 3,4-Dichlor-, 2,3,6-Trichlor-, 4-Fluor-, 4-Chlor-, 4-Brom- und 4-Nitrobenzoesäureamid.

Die Verbindungen der Formel (V) sind bekannt.

Als Verdünnungsmittel kommen praktisch alle inerten organischen Lösungsmittel in Frage. Hierzu gehören insbesondere aliphatische und aromatische, gegebenenfalls halogenierte Kohlenwasserstoffe, wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glykoldimethylether und Diglykoldimethylether, Tetrahydrofuran und Dioxan, Ketone, wie Aceton, Methyl-ethyl-, Methylisopropyl- und Methylisobutylketon, Ester, wie Essigsäuremethylester und -ethylester, Nitrile, wie z.B. Acetonitril und Propionitril, Amide, wie z.B. Dimethylacetamid und N-Methyl-pyrrolidon, sowie Tetramethylensulfon.

Als Katalysatoren können für die Umsetzung gemäss Verfahrensvariante (b) vorzugsweise tertiäre Amine, wie Triethylamin und 1,4-Diazabicyclo[2,2,2]-octan, sowie organische Zinn-Verbindungen, wie z.B. Dibutylzinndilaurat verwendet werden.

Die Reaktionstemperatur kann innerhalb eines grösseren Bereiches variiert werden. Im allgemeinen arbeitet man bei der Verfahrensvariante a) zwischen 20 und 180 °C, vorzugsweise zwischen 40 und 120 °C und bei der Verfahrensvariante b) zwischen 20 und 200 °C, vorzugsweise zwischen 60 und 190 °C. Die erfindungsgemässen Verfahrensvarianten werden im allgemeinen bei Normaldruck durchgeführt.

Zur Durchführung der erfindungsgemässen Verfahrensvarianten werden die Ausgangsstoffe gewöhnlich in etwa äquimolaren Mengen eingesetzt. Ein Überschuss der einen oder anderen Reaktionskomponente bringt keine wesentlichen Vorteile.

Die Aufarbeitung der Reaktionsprodukte geschieht nach üblichen Methoden, z.B. durch Absaugen des ausgefallenen Produktes oder durch Herauslösen von unerwünschten Nebenprodukten aus dem Reaktionsgemisch. Zur Charakterisierung dient der Schmelzpunkt.

Die Wirkstoffe eignen sich bei guter Pflanzenverträglichkeit und günstiger Warmblütertoxizität zur Bekämpfung von tierischen Schädlingen, insbesondere Insekten und Spinnentieren, die in der Landwirtschaft, in Forsten, im Vorrats- und Materialschutz sowie auf dem Hygienesektor vorkommen. Sie sind gegen normal sensible und resistene Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.

Aus der Ordnung der Diplopoda z.B. Blaniulus guttulatus.

Aus der Ordnung der Chilopoda z.B. Geophilus carpophagus, Scutigera spec.

Aus der Ordnung der Symphyla z.B. Scutigerella immaculata.

Aus der Ordnung der Thysanura z.B. Lepisma saccharina.

Aus der Ordnung der Collembola z.B. Onychiurus armatus.

Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.

Aus der Ordnung der Dermaptera z.B. Forficula auricularia.

Aus der Ordnung der Isoptera z.B. Reticulitermes spp.

Aus der Ordnung der Anoplura z.B. Phylloxera vastatrix, Pemphigus spp., Pediculus humanus corporis, Haematopinus spp., Linognathus spp.

Aus der Ordnung der Mallophaga z.B. Trichodectes spp., Damalinea spp.

Aus der Ordnung der Thysanoptera z.B. Hercinothrips femoralis, Thrips tabaci.

Aus der Ordnung der Heteroptera z.B. Eurygaster spp., Dysdercus intermedius, Piesma quadrata, Cimex lectularius, Rhodnius prolixus, Triatoma spp.

Aus der Ordnung der Homoptera z.B. Aleurodes brassicae, Bemisia tabaci, Trialeurodes vaporariorum, Aphis gossypii, Brevicoryne brassicae, Cryptomyzus ribis, Doralis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.

Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella,

Agrotis spp., Euxoa spp., Feltia spp., Earias insulana, Heliothis spp., Laphygma exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.

Aus der Ordnung der Coleoptera z.B. Anobium punctatum, Rhizopertha dominica, Bruchidius obtectus, Acanthoscelides obtectus, Hylotrupes bajules, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.

Aus der Ordnung der Hymenoptera z.B. Diprion spp., Hoplocampa spp., Lasius spp., Monomorium pharaonis, Vespa spp.

Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp.

Aus der Ordnung der Arachnida z.B. Scorpio maurus, Latrodectus mactans.

Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp.,

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgas, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnussschalen, Maiskolben und Tabakstengel; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweisshydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90%.

Die erfindungsgemässen Wirkstoffe können in ihren handelsüblichen Formulierungen sowie in den aus diesen Formulierungen bereiteten Anwendungsformen in Mischung mit anderen Wirkstoffen, wie Insektiziden, Lockstoffen, Sterilantien, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a.

Die erfindungsgemässen Wirkstoffe können ferner in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne dass der zugesetzte Synergist selbst aktiv wirksam sein muss.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepassten üblichen Weise.

Bei der Anwendung gegen Hygiene- und Vorratsschädlinge zeichnen sich die Wirksstoffe durch eine hervorragende Residualwirkung auf Holz und Ton sowie durch eine gute Alkalistabilität auf gekälkten Unterlagen aus.

Die erfindungsgemässen Wirkstoffe eignen sich auch zur Bekämpfung von Ekto- und Endoparasiten auf dem Gebiet der Tierhaltung und Viehzucht, wobei durch die Bekämpfung der Schädlinge bessere Ergebnisse, z.B. höhere Milchleistungen, höheres Gewicht, schöneres Tierfell, längere Lebensdauer usw. erreicht werden können.

Die Anwendung der erfindungsgemässen Wirkstoffe geschieht auf diesen Gebieten in bekannter Weise, wie durch äusserliche Anwendung in Form beispielsweise des Tauchens (Dippen), Sprühens (Sprayen), Aufgiessens (pour-on and spot-on) und das Einpudern sowie durch orale Anwendung, beispielsweise über das Futter oder Trinkwasser in Form von beispielsweise Tabletten, Kapseln, Tränken, Granulaten.

Die Herstellung der erfindungsgemässen Verbindungen soll durch die folgenden Herstellungsbeispiele erläutert werden:

Beispiel 1

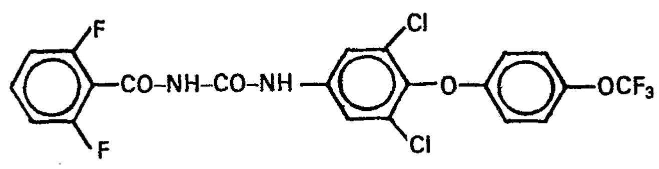

(Verfahrensvariante a)

Zu einer Lösung von 6,76 g (0,02 Mol) 3,5-Dichlor-4-(4-trifluormethoxy-phenoxy)-anilin in 60 ml trockenem Toluol werden bei 60 °C 3,66 g (0,02 Mol) 2,6-Difluorbenzoylisocyanat in 10 ml Toluol gegeben. Das Gemisch wird anschliessend eine Stunde bei 80 °C gerührt. Nach Abkühlung auf 20 °C wird abgesaugt und getrocknet.

Man erhält 9 g (86,5% der Theorie) 1-(2,6-Difluorbenzoyl)-3- [3,5-dichlor-4- (4-trifluormethoxy-phenoxy)- phenyl]-harnstoff mit einem Schmelzpunkt Fp. 194 °C.

Beispiel 2

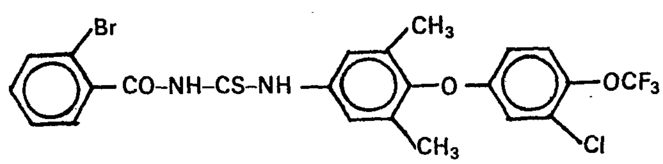

(Verfahrensvariante b)

Zu einer Lösung von 3,32 g (0,01 Mol) 3,5-Dimethyl-4- (3-chlor-4-trifluormethoxy-phenoxy)-anilin in 50 ml trockenem Toluol werden bei 60 °C 2,42 g (0,01 Mol) 2-Brombenzoylisothiocyanat in 10 ml Toluol gegeben. Das Gemisch wird anschliessend 30 Minuten bei 80 °C gerührt und dann auf ein Volumen von ca. 20 ml eingeengt. Der Niederschlag wird abgesaugt und getrocknet.

Man erhält 5,1 g (89% der Theorie) 1-(2-Brombenzoyl)-3- [3,5-dimethyl-4- (3-chlor-4-trifluormethoxy-phenoxy)-phenyl]-harnstoff mit einem Schmelzpunkt Fp.: 167 °C.

Analog Beispiele 1 oder 2 bzw. Verfahrensvariante (a) oder (b) wurden folgende Verbindungen der Formel (I) hergestellt:

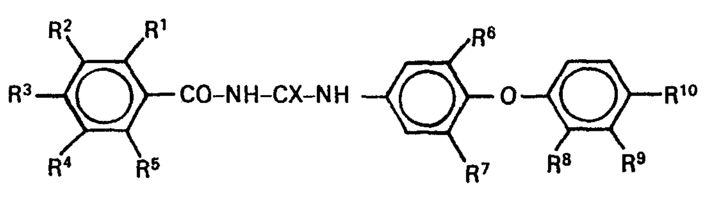

(I)

Tabelle 2

| Beisp. Nr. | $R^1$ | $R^2$ | $R^3$ | $R^4$ | $R^5$ | $R^6$ | $R^7$ | $R^8$ | $R^9$ | $R^{10}$ | X | Fp. °C |
|---|---|---|---|---|---|---|---|---|---|---|---|---|
| 3 | Cl | H | H | H | H | Cl | Cl | H | H | $OCF_3$ | O | 194 |
| 4 | Cl | H | H | H | F | Cl | Cl | H | H | $OCF_3$ | O | 193 |
| 5 | Br | H | H | H | H | Cl | Cl | H | H | $SCF_3$ | O | 199 |
| 6 | Cl | H | H | H | H | Cl | Cl | H | H | $SCF_3$ | O | 198 |
| 7 | F | H | H | H | F | Cl | Cl | H | H | $SCF_3$ | O | 215 |
| 8 | Cl | H | H | H | Cl | Cl | Cl | H | H | $SCF_3$ | O | 226 |
| 9 | Cl | H | H | H | F | Cl | Cl | H | H | $SCF_3$ | O | 226 |
| 10 | F | H | H | H | F | Cl | Cl | Cl | H | $OCF_3$ | O | 167 |
| 11 | Cl | H | H | H | F | Cl | Cl | Cl | H | $OCF_3$ | O | 154 |
| 12 | Cl | H | H | H | H | Cl | Cl | Cl | H | $OCF_3$ | O | 178 |
| 13 | F | H | H | H | F | Cl | Cl | Cl | H | $OCF_3$ | S | 170 |
| 14 | Br | H | H | H | H | Cl | Cl | Cl | H | $OCF_3$ | S | 151 |
| 15 | Cl | H | H | H | F | Cl | Cl | Cl | H | $OCF_3$ | S | 182 |
| 16 | $NO_2$ | H | H | H | H | Cl | Cl | Cl | H | $OCF_3$ | O | 202 |
| 17 | $SCH_3$ | H | H | H | H | Cl | Cl | Cl | H | $OCF_3$ | O | 181 |
| 18 | H | H | Cl | H | H | Cl | Cl | Cl | H | $OCF_3$ | O | 232 |
| 19 | Cl | H | $NO_2$ | H | H | Cl | Cl | Cl | H | $OCF_3$ | O | 230 |
| 20 | H | Cl | Cl | H | H | Cl | Cl | Cl | H | $OCF_3$ | O | 128 |
| 21 | Br | H | H | H | H | Cl | Cl | Cl | H | $OCF_3$ | O | 171 |
| 22 | $CH_3$ | H | H | H | H | Cl | Cl | Cl | H | $OCF_3$ | O | 188 |
| 23 | Cl | H | H | H | Cl | Cl | Cl | Cl | H | $OCF_3$ | O | 203 |
| 24 | $CH_3$ | H | H | H | H | Cl | Cl | H | H | $OCF_3$ | O | 206 |
| 25 | Br | H | H | H | H | Cl | Cl | H | H | $OCF_3$ | O | 200 |
| 26 | Cl | H | H | H | Cl | Cl | Cl | H | H | $OCF_3$ | S | 194 |
| 27 | Cl | H | H | H | F | Cl | Cl | H | H | $OCF_3$ | S | 152 |
| 28 | F | H | H | H | F | Cl | Cl | H | H | $OCF_3$ | S | 145 |
| 29 | Cl | H | H | H | Cl | H | H | H | H | $OCF_3$ | S | 154 |
| 30 | F | H | H | H | F | H | H | H | H | $OCF_3$ | S | 139 |
| 31 | Cl | H | H | H | F | Cl | H | H | H | $OCF_3$ | S | 121 |
| 32 | Cl | H | H | H | H | H | H | Cl | H | $OCF_3$ | O | 154 |
| 33 | F | H | H | H | F | H | H | Cl | H | $OCF_3$ | O | 162 |
| 34 | Br | H | H | H | H | H | H | Cl | H | $OCF_3$ | O | 167 |
| 35 | Cl | H | H | H | Cl | H | H | Cl | H | $OCF_3$ | S | 156 |
| 36 | Cl | H | H | H | F | H | H | Cl | H | $OCF_3$ | S | 148 |
| 37 | Cl | H | H | H | H | Cl | H | Cl | H | $OCF_3$ | O | 151 |
| 38 | F | H | H | H | F | Cl | H | Cl | H | $OCF_3$ | O | 156 |
| 39 | Cl | H | H | H | Cl | Cl | H | Cl | H | $OCF_3$ | S | 143 |
| 40 | Cl | H | H | H | Cl | Cl | H | Cl | H | $OCF_3$ | O | 188 |
| 41 | Cl | H | H | H | H | Cl | Cl | H | Cl | $OCF_3$ | O | 200 |
| 42 | Cl | H | H | H | H | Cl | Cl | Cl | H | $SCF_3$ | O | 207 |
| 43 | F | H | H | H | F | Cl | Cl | Cl | H | $SCF_3$ | O | 216 |
| 44 | F | H | H | H | F | Cl | Cl | H | Cl | $OCF_3$ | O | 196 |
| 45 | Cl | H | H | H | F | Cl | Cl | Cl | H | $SCF_3$ | S | 166 |
| 46 | $CH_3$ | H | H | H | H | Cl | Cl | Cl | H | $SCF_3$ | O | 207 |
| 47 | Cl | H | H | H | F | Cl | Cl | [illegible] | Cl | $OCF_3$ | S | 161 |
| 48 | Br | H | H | H | H | Cl | Cl | H | Cl | $OCF_3$ | S | 154 |
| 49 | Br | H | H | H | H | Cl | Cl | Cl | H | $SCF_3$ | O | 204 |
| 50 | Br | H | H | H | H | Cl | Cl | H | Cl | $OCF_3$ | O | 180 |
| 51 | $CH_3$ | H | H | H | H | Cl | Cl | H | Cl | $OCF_3$ | O | 207 |
| 52 | Cl | H | H | H | Cl | Cl | Cl | Cl | H | $SCF_3$ | O | 205 |
| 53 | Cl | H | H | H | Cl | Cl | Cl | Cl | H | $OCF_3$ | O | 220 |
| 54 | Cl | H | H | H | Cl | $CH_3$ | $CH_3$ | H | Cl | $OCF_3$ | O | 191–192 |
| 55 | Cl | H | H | H | H | $CH_3$ | $CH_3$ | H | Cl | $OCF_3$ | O | 174–175 |
| 56 | Cl | H | H | H | F | $CH_3$ | $CH_3$ | H | Cl | $OCF_3$ | O | 172–173 |
| 57 | F | H | H | H | F | $CH_3$ | $CH_3$ | H | Cl | $OCF_3$ | O | 182–183 |
| 58 | Cl | H | H | H | H | $CH_3$ | $CH_3$ | Cl | H | $OCF_3$ | O | 200–206 |
| 59 | Cl | H | H | H | H | $CH_3$ | $CH_3$ | H | H | $SCF_3$ | O | 201 |
| 60 | Cl | H | H | H | F | $CH_3$ | $CH_3$ | Cl | H | $OCF_3$ | O | 151 |
| 61 | F | H | H | H | F | $CH_3$ | $CH_3$ | H | H | $SCF_3$ | O | 200 |

Tabelle 2

| Beisp. Nr. | $R^1$ | $R^2$ | $R^3$ | $R^4$ | $R^5$ | $R^6$ | $R^7$ | $R^8$ | $R^9$ | $R^{10}$ | X | Fp. °C |
|---|---|---|---|---|---|---|---|---|---|---|---|---|
| 62 | F | F | F | F | F | Cl | Cl | Cl | H | $OCF_3$ | O | 196 |
| 63 | F | H | H | H | F | $CH_3$ | $CH_3$ | Cl | H | $OCF_3$ | O | 149 |
| 64 | Cl | H | H | H | F | $CH_3$ | $CH_3$ | Cl | H | $OCF_3$ | S | 155 |
| 65 | F | H | H | H | F | $CH_3$ | $CH_3$ | Cl | H | $OCF_3$ | S | 133 |

Die biologische Wirksamkeit der erfindungsgemässen Verbindungen soll anhand der folgenden Beispiele erläutert werden.

Beispiel A
Plutella-Test

Lösungsmittel: 15 Gewichtsteile Dimethylformamid

Emulgator: 1 Gewichtsteil Alkylarylpolyglykolether

Zur Herstellung einer zweckmässigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Raupen der Kohlschabe (Plutella maculipennis) besetzt, solange die Blätter noch feucht sind.

Nach der gewünschten Zeit wird die Abtötung in % bestimmt. Dabei bedeutet 100%, dass alle Raupen abgetötet wurden; 0% bedeutet, dass keine Raupen abgetötet wurden.

Bei diesem Test zeigten z.B. bei einer Konzentration von 0,001% beispielsweise die Verbindungen der Herstellungsbeispiele 1, 3, 4, 7, 10, 11, 12, 21, 22, 37, 38, 43, 44, 57, 58, 60, 61 und 63 nach 7 Tagen eine Abtötung von 100%.

Beispiel B
Mückenlarven-Test
Testtiere: Aedes aegypti (2. Larvenstadium)

Lösungsmittel: Aceton: 99 Gewichtsteile

Emulgator: Alkylarylpolyglykolether: 1 Gewichtsteil

Zur Herstellung einer zweckmässigen Wirkstoffzubereitung löst man 2 Gew.-Teile Wirkstoff in 1000 Volumenteilen Lösungsmittel, das Emulgator in der oben angegebenen Menge enthält. Die so erhaltene Lösung wird mit Wasser auf die gewünschten geringeren Konzentrationen verdünnt.

Man füllt die wässrigen Wirkstoffzubereitungen der gewünschten Konzentration in Kunststoffbecher und setzt anschliessend 20 Mückenlarven (2. Larvenstadium) in jeden Becher ein. Die Larven werden täglich gefüttert.

Nach 1, 8 und 21 Tagen wird der Abtötungsgrad in % bestimmt. Dabei bedeutet 100%, dass alle Larven bzw. Puppen abgetötet worden sind. 0% bedeutet, dass überhaupt keine Larven abgetötet worden sind.

Bei diesem Test zeigten z.B. bei einer Konzentration von $10^{-4}$ ppm beispielsweise die Verbindung der Herstellungsbeispiele 38 und 63 nach 21 Tagen eine Abtötung von 100%.

Beispiel C
Test mit parasitierenden Fliegenlarven (Lucilia cuprina)

Lösungsmittel: 35 Gew.-Teile Ethylenglykolmonomethylether

Emulgator: 35 Gew.- Teile Nonylphenolpolyglykolether.

Zur Herstellung einer zweckmässigen Wirkstoffzubereitung vermischt man 30 Gew.-Teile der betreffenden aktiven Substanz mit der angegebenen Menge Lösungsmittel, das den obengenannten Anteil Emulgator enthält und verdünnt das so erhaltene Konzentrat mit Wasser auf die gewünschte Konzentration.

Etwa 20 Fliegenlarven (Lucilia cuprina) werden in ein Teströhrchen gebracht, welches ca. 2 $cm^3$ Pferdemuskulatur enthält. Auf dieses Pferdefleisch werden 0,5 ml der Wirkstoffzubereitung gebracht. Nach 24 Stunden wird der Abtötungsgrad in % bestimmt. Dabei bedeuten 100%, dass alle, und 0% (Kontrolle), dass keine Larven abgetötet worden sind.

Bei einem Rest z.B. mit einer Wirkstoffkonzentration von 1000 ppm zeigten beispielsweise die Verbindungen der Herstellungsbeispiele (1) und (4) eine 100%ige Abtötung.

Beispiel D
Tetranychus-Test (resistent)

Lösungsmittel: 3 Gewichtsteile Dimethylformamid

Emulgator: 1 Gewichtsteil Alkylarylpolyglykolether

Zur Herstellung einer zweckmässigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Bohnenpflanzen (Phaseolus vulgaris), die stark von allen Entwicklungsstadien der gemeinen Spinnmilbe oder Bohnenspinnmilbe (Tetranychus urticae) befallen sind, werden durch Tauchen in die Wirkstoffzubereitung der gewünschten Konzentration behandelt.

Nach der gewünschten Zeit wird die Abtötung in % bestimmt. Dabei bedeutet 100%, dass alle Spinnmilben abgetötet wurden; 0% bedeutet, dass keine Spinnmilben abgetötet wurde.

Bei diesem Test zeigten, z.B. bei einer Konzentration von 0,1% die Verbindungen der Herstellungsbeispiele 2, 33, 37, 38, 43, 47, 55, 56, 57, 58, 60, 61 und 63 nach 10 Tagen eine Abtötung von 98%.

**Patentansprüche**

1. 1-Phenyl-3-benzoyl-(thio)harnstoffe der Formel (I)

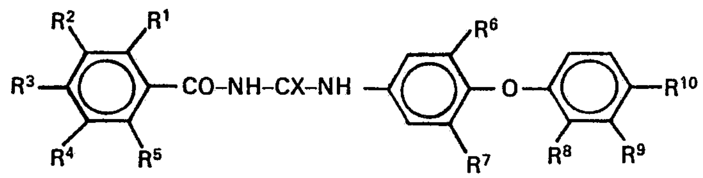

(I)

in welcher

X für Schwefel oder Sauerstoff steht,

$R^1$ für Wasserstoff, Halogen, Nitro oder für einen Rest aus der Reihe $C_1$–$C_6$-Alkyl und $C_1$–$C_6$-Alkylthio steht,

$R^2$, $R^4$ und $R^5$ gleich oder verschieden sind und für Wasserstoff oder Halogen stehen,

$R^3$ für Wasserstoff, Halogen oder Nitro steht,

$R^6$ und $R^7$ gleich oder verschieden sind und für Wasserstoff, Halogen oder für einen $C_1$–$C_6$-Alkyl-Rest stehen,

$R^8$ und $R^9$ gleich oder verschieden sind und für Wasserstoff oder Halogen stehen und

$R^{10}$ für Chlordifluormethylthio, Trifluormethylthio, Chlordifluormethoxy oder Trifluormethoxy steht, wobei

(1) wenn X für Sauerstoff steht und

$R^2$, $R^3$, $R^4$, $R^6$, $R^8$ und $R^9$ für Wasserstoff stehen und

$R^7$ für Wasserstoff oder Halogen steht und

(a) $R^1$ und $R^5$ beide für Halogen stehen oder

(b) $R^1$ für Halogen, Nitro oder $C_1$–$C_6$-Alkyl und $R^5$ für Wasserstoff steht, in diesen Fällen den Rest

$R^{10}$ stets Chlordifluormethylthio oder Trifluormethylthio bedeutet und wobei

(2) wenn X für Sauerstoff oder Schwefel steht und

$R^6$ für Chlor steht und

$R^8$ für Wasserstoff oder Chlor steht und

$R^9$ für Wasserstoff steht und

$R^{10}$ für Chlordifluormethylthio, Trifluormethylthio, Chlordifluormethoxy oder Trifluormethoxy steht und

(a) $R^1$ und $R^3$ für Halogen und $R^2$, $R^4$ und $R^5$ für Wasserstoff stehen oder

(b) $R^5$ und $R^3$ für Halogen und $R^1$, $R^2$ und $R^4$ für Wasserstoff stehen oder

(c) $R^1$ und $R^4$ für Halogen und $R^2$, $R^3$ und $R^5$ für Wasserstoff stehen oder

(d) $R^2$ und $R^5$ für Halogen und $R^1$, $R^3$ und $R^4$ für Wasserstoff stehen, in diesen Fällen den Rest

$R^7$ stets Wasserstoff oder $C_1$–$C_6$-Alkyl bedeutet.

2. Verbindungen der Formel (I) gemäss Anspruch 1, in welcher

X für Sauerstoff oder Schwefel steht,

$R^1$ für Wasserstoff, Fluor, Chlor, Brom, Nitro, Methyl, Ethyl, n-Propyl, i-Propyl, n-Butyl, i-Butyl, sec.-Butyl, tert.-Butyl, Methylthio, Ethylthio, n-Propylthio, i-Propylthio, n-Butylthio, i-Butylthio, sec.-Butylthio oder tert.-Butylthio steht,

$R^2$, $R^4$ und $R^5$ gleich oder verschieden sind und für Wasserstoff, Fluor, Chlor oder Brom stehen,

$R^3$ für Wasserstoff, Fluor, Chlor, Brom oder Nitro steht,

$R^6$ und $R^7$ gleich oder verschieden sind und für Wasserstoff, Fluor, Chlor, Brom, Methyl, Ethyl, n-Propyl, i-Propyl, n-Butyl, i-Butyl, sec.-Butyl oder tert.-Butyl stehen,

$R^8$ und $R^9$ gleich oder verschieden sind und für Wasserstoff, Fluor, Chlor oder Brom stehen und

$R^{10}$ für Trifluormethylthio oder Trifluormethoxy steht, wobei

(1) wenn X für Sauerstoff steht und

$R^2$, $R^3$, $R^4$, $R^6$, $R^8$ und $R^9$ für Wasserstoff stehen und

$R^7$ für Wasserstoff, Fluor, Chlor oder Brom steht,

(a) $R^1$ und $R^5$ beide für Fluor, Chlor und/oder Brom stehen oder

(b) $R^1$ für Fluor, Chlor, Brom, Nitro oder die oben genannten Alkylreste und $R^5$ für Wasserstoff steht, in diesen Fällen den Rest

$R^{10}$ stets Trifluormethylthio bedeutet und

$R^2$, $R^4$ und $R^5$ für Wasserstoff, Fluor, Chlor oder Brom stehen,

$R^3$ für Wasserstoff, Fluor, Chlor oder Nitro steht,

$R^6$ und $R^7$ gleich oder verschieden sind und für Wasserstoff, Chlor oder Methyl stehen,

$R^8$ und $R^9$ gleich oder verschieden sind und für Wasserstoff, Fluor oder Chlor stehen, und

$R^{10}$ für Trifluormethylthio oder Trifluormethoxy steht, wobei

(1) wenn

$R^2$, $R^3$, $R^4$, $R^6$, $R^8$ und $R^9$ für Wasserstoff stehen und

$R^7$ für Wasserstoff oder Chlor steht und

(a) $R^1$ und $R^5$ beide für Fluor, Chlor und/oder Brom stehen oder

(b) $R^1$ für Fluor, Chlor, Brom, Nitro oder Methyl und $R^5$ für Wasserstoff steht, in diesen Fällen der Rest

$R^{10}$ stets Trifluormethylthio bedeutet und wobei

(2) wenn X für Sauerstoff oder Schwefel steht und

$R^6$ für Chlor steht und
$R^8$ für Wasserstoff oder Chlor steht und
$R^9$ für Wasserstoff steht und
$R^{10}$ für Trifluormethylthio oder Trifluormethoxy steht und
(a) $R^1$ und $R^3$ für Fluor, Chlor oder Brom und $R^2$, $R^4$ und $R^5$ für Wasserstoff stehen oder
(b) $R^5$ und $R^3$ für Fluor, Chlor oder Brom und $R^1$, $R^2$ und $R^4$ für Wasserstoff stehen oder
(c) $R^1$ und $R^4$ für Fluor, Chlor oder Brom und $R^2$, $R^3$ und $R^5$ für Wasserstoff stehen oder
(d) $R^2$ und $R^5$ für Fluor, Chlor oder Brom und $R^1$, $R^3$ und $R^4$ für Wasserstoff stehen, in diesen Fällen der Rest
$R^7$ stets Wasserstoff oder die oben genannten Alkylreste bedeutet.

3. Verbindungen gemäss den Ansprüchen 1 und 2, in welchen X der Formel I gemäss Anspruch 1 für Sauerstoff steht.

4. Verbindungen der Formel (I) gemäss Anspruch 1, in welcher
X für Sauerstoff steht,
$R^1$ für Wasserstoff, Fluor, Chlor, Brom, Nitro, Methyl oder Methylthio steht,
$R^6$ für Chlor steht und
$R^8$ für Wasserstoff oder Chlor steht und
$R^9$ für Wasserstoff steht und
$R^{10}$ für Trifluormethylthio oder Trifluormethoxy steht und
(a) $R^1$ und $R^3$ für Fluor, Chlor und/oder Brom und $R^2$, $R^4$ und $R^5$ für Wasserstoff stehen oder
(b) $R^5$ und $R^3$ für Fluor, Chlor und/oder Brom und $R^1$, $R^2$ und $R^4$ für Wasserstoff stehen oder
(c) $R^1$ und $R^4$ für Fluor, Chlor und/oder Brom und $R^2$, $R^3$ und $R^5$ für Wasserstoff stehen und
(d) $R^2$ und $R^5$ für Fluor, Chlor und/oder Brom und $R^1$, $R^3$ und $R^4$ für Wasserstoff stehen, in diesen Fällen der Rest
$R^7$ stets Wasserstoff oder Methyl bedeutet.

5. Verfahren zur Herstellung von 1-Phenyl-3-benzoyl-(thio)harnstoffen der Formel (I)

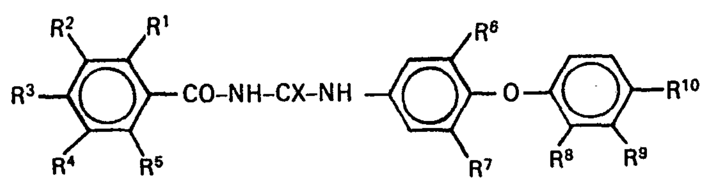

(I)

in welcher
X für Schwefel oder Sauerstoff steht,
$R^1$ für Wasserstoff, Halogen, Nitro oder für einen Rest aus der Reihe $C_1$–$C_6$-Alkyl und $C_1$–$C_6$-Alkylthio steht,
$R^2$, $R^4$ und $R^5$ gleich oder verschieden sind und für Wasserstoff oder Halogen stehen,
$R^3$ für Wasserstoff, Halogen oder Nitro steht,
$R^6$ und $R^7$ gleich oder verschieden sind und für Wasserstoff, Halogen oder für einen $C_1$–$C_6$-Alkyl-Rest stehen,
$R^8$ und $R^9$ gleich oder verschieden sind und für Wasserstoff oder Halogen stehen und
$R^{10}$ für Chlordifluormethylthio, Trifluormethylthio, Chlordifluormethoxy oder Trifluormethoxy steht, wobei
(1) wenn X für Sauerstoff steht und
$R^2$, $R^3$, $R^4$, $R^6$, $R^8$ und $R^9$ für Wasserstoff stehen und
$R^7$ für Wasserstoff oder Halogen steht und
(a) $R^1$ und $R^5$ beide für Halogen stehen oder
(b) $R^1$ für Halogen, Nitro oder $C_1$–$C_6$-Alkyl und $R^5$ für Wasserstoff steht, in diesen Fällen den Rest
$R^{10}$ stets Chlordifluormethylthio oder Trifluormethylthio bedeutet und wobei
(2) wenn X für Sauerstoff oder Schwefel steht und
$R^6$ für Chlor steht und
$R^8$ für Wasserstoff oder Chlor steht und
$R^9$ für Wasserstoff steht und
$R^{10}$ für Chlordifluormethylthio, Trifluormethylthio, Chlordifluormethoxy oder Trifluormethoxy steht und
(a) $R^1$ und $R^3$ für Halogen und $R^2$, $R^4$ und $R^5$ für Wasserstoff stehen oder
(b) $R^5$ und $R^3$ für Halogen und $R^1$, $R^2$ und $R^4$ für Wasserstoff stehen oder
(c) $R^1$ und $R^4$ für Halogen und $R^2$, $R^3$ und $R^5$ für Wasserstoff stehen oder
(d) $R^2$ und $R^5$ für Halogen und $R^1$, $R^3$ und $R^4$ für Wasserstoff stehen, in diesen Fällen den Rest
$R^7$ stets Wasserstoff oder $C_1$–$C_6$-Alkyl bedeutet, dadurch gekennzeichnet, dass man
a) substituierte Aniline der Formel (II),

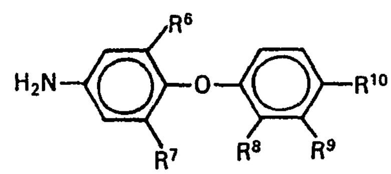

(II)

in welcher
$R^6$, $R^7$, $R^8$, $R^9$ und $R^{10}$ die oben angegebenen Bedeutungen haben, mit Benzoyl-iso(thio)cyanaten der Formel (III),

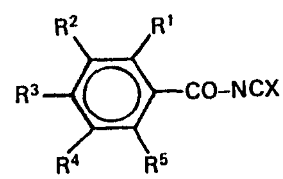

(III)

in welcher
X, $R^1$, $R^2$, $R^3$, $R^4$, und $R^5$ die oben angegebenen Bedeutungen haben, gegebenenfalls in Gegenwart eines Verdünnungsmittels umsetzt oder
b) substituierte Phenyl-iso(thio)cyanate der Formel (IV),

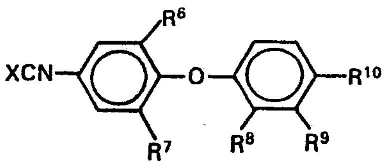

(IV)

in welcher

X, $R^6$, $R^7$, $R^8$, $R^9$ und $R^{10}$ die oben angegebene Bedeutung haben, mit Benzoesäureamiden der Formel (V),

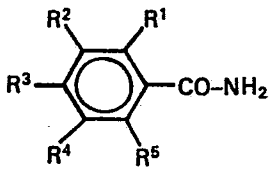

(V)

in welcher

$R^1$, $R^2$, $R^3$, $R^4$ und $R^5$ die oben angegebene Bedeutung haben, gegebenenfalls in Gegenwart eines Katalysators und gegebenenfalls in Gegenwart eines Verdünnungsmittels umsetzt.

6. Schädlingsbekämpfungsmittel, gekennzeichnet, durch einen Gehalt an mindestens einer Verbindung der Formel I gemäss den Ansprüchen 1 oder 5.

7. Verwendung von Verbindungen der Formel I gemäss den Ansprüchen 1 oder 5 zur Bekämpfung von Schädlingen, insbesondere Insekten und Spinnentieren.

8. Verfahren zur Bekämpfung von Schädlingen, dadurch gekennzeichnet, dass man Verbindungen der Formel I gemäss den Ansprüchen 1 oder 5 auf die Schädlinge, vorzugsweise Insekten oder Spinnentiere oder ihren Lebensraum einwirken lässt.

9. Verfahren zur Herstellung von Schädlingsbekämpfungsmitteln, dadurch gekennzeichnet, dass man Verbindungen der Formel I gemäss den Ansprüchen 1 oder 5 mit Streckmitteln und/oder oberflächenaktiven Mitteln vermischt.

**Revendications**

1. 1-phényl-3-benzoyl-(thio)urées de formule I

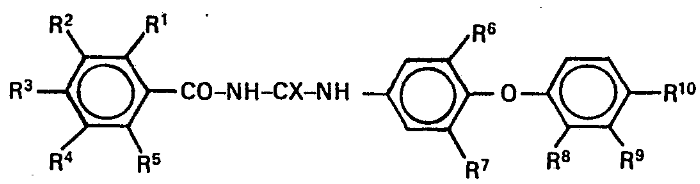

(I)

dans laquelle

X représente le soufre ou l'oxygène,

$R^1$ représente l'hydrogène, un halogène, un groupe nitro ou un groupe de la classe des groupes alkyle en $C_1$–$C_6$ et alkylthio en $C_1$–$C_6$,

$R^2$, $R^4$ et $R^5$, ayant des significations identiques ou différentes, représentent chacun l'hydrogène ou un halogène,

$R^3$ représente l'hydrogène, un halogène ou un groupe nitro,

$R^6$ et $R^7$, ayant des significations identiques ou différentes, représentent chacun l'hydrogène, un halogène ou un groupe alkyle en $C_1$–$C_6$,

$R^8$ et $R^9$, ayant des significations identiques ou différentes, représentent chacun l'hydrogène ou un halogène, et

$R^{10}$ représente un groupe chlorodifluorométhylthio, trifluorométhylthio, chlorodifluorométhoxy ou trifluorométhoxy, avec les conditions supplémentaires suivantes:

(1) lorsque X représente l'oxygène et

$R^2$, $R^3$, $R^4$, $R^6$, $R^8$ et $R^9$ l'hydrogène, et

$R^7$ l'hydrogène ou un halogène, et que

(a) $R^1$ et $R^5$ représentent tous deux des halogènes, ou bien

(b) $R^1$ représente un halogène, un groupe nitro ou alkyle en $C_1$–$C_6$ et $R^5$ l'hydrogène, dans de tels cas,

$R^{10}$ représente toujours un groupe chlorodifluorométhylthio ou trifluorométhylthio, et

(2) lorsque X représente l'oxygène ou le soufre, et $R^6$ le chlore, et

$R^8$ l'hydrogène ou le chlore, et

$R^9$ l'hydrogène, et

$R^{10}$ un groupe chlorodifluorométhylthio, trifluorométhylthio, chlorodifluorométhoxy ou trifluorométhoxy, et que

(a) $R^1$ et $R^3$ représentent des halogènes et $R^2$, $R^4$ et $R^5$ l'hydrogène, ou bien

(b) $R^5$ et $R^3$ représentent des halogènes et $R^1$, $R^2$ et $R^4$ l'hydrogène, ou bien

(c) $R^1$ et $R^4$ représentent des halogènes et $R^2$, $R^3$ et $R^5$ l'hydrogène, ou bien

(d) $R^2$ et $R^5$ représentent des halogènes et $R^1$, $R^3$ et $R^4$ l'hydrogène, dans de tels cas,

$R^7$ représente toujours l'hydrogène ou un groupe alkyle en $C_1$–$C_6$.

2. Composés de formule I selon la revendication 1, dans laquelle:

X représente l'oxygène ou le soufre,

$R^1$ représente l'hydrogène, le fluor, le chlore, le brome, un groupe nitro, méthyle, éthyle, n-propyle, isopropyle, n-butyle, isobutyle, sec.-butyle, tert.-butyle, méthylthio, éthylthio, n-propylthio, isopropylthio, n-butylthio, isobutylthio, sec.-butylthio ou tert.-butylthio,

$R^2$, $R^4$ et $R^5$, ayant des significations identiques ou différentes, représentent chacun l'hydrogène, le fluor, le chlore ou le brome, $R^3$ représente l'hydrogène, le fluor, le chlore, le brome ou un groupe nitro,

$R^6$ et $R^7$, ayant des significations identiques ou différentes, représentent chacun l'hydrogène, le fluor, le chlore, le brome, un groupe méthyle, éthyle, n-propyle, isopropyle, n-butyle, isobutyle, sec.-butyle ou tert.-butyle,

$R^8$ et $R^9$, ayant des significations identiques ou différentes, représentent chacun l'hydrogène, le fluor, le chlore ou le brome, et

$R^{10}$ représente un groupe trifluorométhylthio ou trifluorométhoxy, avec les conditions supplémentaires suivantes:

(1) lorsque X représente l'oxygène, et

$R^2$, $R^3$, $R^4$, $R^6$, $R^8$ et $R^9$ représentent l'hydrogène, et

$R^7$ l'hydrogène, le fluor, le chlore ou le brome, et que

(a) $R^1$ et $R^5$ représentent tous deux le fluor, le chlore et/ou le brome, ou bien

(b) $R^1$ représente le fluor, le chlore, le brome, un groupe nitro ou les groupes alkyle mentionnés ci-dessus et

$R^5$ l'hydrogène, dans de tels cas,

$R^{10}$ représente toujours un groupe trifluorométhylthio, et

(2) lorsque X représente l'oxygène ou le soufre, et

$R^6$ le chlore, et

$R^8$ l'hydrogène ou le chlore, et

$R^9$ l'hydrogène, et que

$R^{10}$ représente un groupe trifluorométhylthio ou trifluorométhoxy, et que

(a) $R^1$ et $R^3$ représentent le fluor, le chlore ou le brome et $R^2$, $R^4$ et $R^5$ l'hydrogène, ou bien

(b) $R^5$ et $R^3$ représentent le fluor, le chlore ou le brome et $R^1$, $R^2$ et $R^4$ l'hydrogène, ou bien

(c) $R^1$ et $R^4$ représentent le fluor, le chlore ou le brome, et $R^2$, $R^3$ et $R^5$ représentent l'hydrogène, ou bien

(d) $R^2$ et $R^5$ représentent le fluor, le chlore ou le brome, et $R^1$, $R^3$ et $R^4$ l'hydrogène, dans de tels cas,

$R^7$ représente toujours l'hydrogène ou les groupes alkyle mentionnés ci-dessus.

3. Composés selon les revendications 1 et 2, dans lesquels X de la formule I de la revendication 1 représente l'oxygène.

4. Composés de formule I selon la revendication 1, dans lesquels;

X représente l'oxygène, et

$R^1$ représente l'hydrogène, le fluor, le chlore, le brome, un groupe nitro, méthyle ou méthylthio,

$R^2$, $R^4$ et $R^5$ représentent l'hydrogène, le fluor, le chlore ou le brome,

$R^3$ représente l'hydrogène, le fluor, le chlore ou un groupe nitro,

$R^6$ et $R^7$, ayant des significations identiques ou différentes, représentent chacun l'hydrogène, le chlore ou un groupe méthyle,

$R^8$ et $R^9$, ayant des significations identiques ou différentes, représentent chacun l'hydrogène, le fluor ou le chlore, et

$R^{10}$ représente un groupe trifluorométhylthio ou trifluorométhoxy, avec les conditions supplémentaires suivantes:

(1) lorsque $R^2$, $R^3$, $R^4$, $R^6$, $R^8$ et $R^9$ représentent l'hydrogène, et

$R^7$ l'hydrogène ou le chlore, et que

(a) $R^1$ et $R^5$ représentent tous deux le fluor, le chlore et/ou le brome, ou bien

(b) $R^1$ représente le fluor, le chlore, le brome, un groupe nitro ou méthyle et

$R^5$ l'hydrogène, dans de tels cas,

$R^{10}$ représente toujours un groupe trifluorométhylthio, et (2) lorsque

$R^6$ représente le chlore, et

$R^8$ l'hydrogène ou le chlore, et

$R^9$ l'hydrogène, et

$R^{10}$ un groupe trifluorométhylthio ou trifluorométhoxy, et que

(a) $R^1$ et $R^3$ représentent le fluor, le chlore et/ou le brome, et $R^2$, $R^4$ et $R^5$ l'hydrogène, ou bien

(b) $R^5$ et $R^3$ représentent le fluor, le chlore et/ou le brome, et $R^1$, $R^2$ et $R^4$ l'hydrogène, ou bien

(c) $R^1$ et $R^4$ représentent le fluor, le chlore et/ou le brome, et $R^2$, $R^3$ et $R^5$ l'hydrogène, et

(d) $R^2$ et $R^5$ représentent le fluor, le chlore et/ou le brome, et $R^1$, $R^3$ et $R^4$ l'hydrogène, dans de tels cas,

$R^7$ représente toujours l'hydrogène ou un groupe méthyle.

5. Procédé de préparation des 1-phényl-3-benzoyl(thio)urées de formule I:

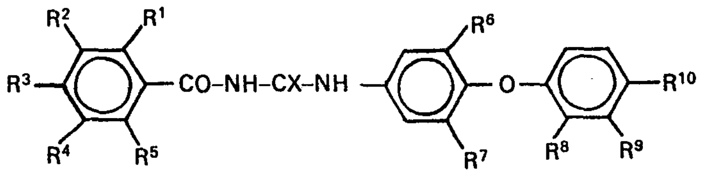

(I)

dans laquelle

X représente le soufre ou l'oxygène,

$R^1$ représente l'hydrogène, un halogène, un groupe nitro ou un groupe de la classe des groupes alkyle en $C_1$–$C_6$ ou alkylthio en $C_1$–$C_6$,

$R^2$, $R^4$ et $R^5$, ayant des significations identiques ou différentes, représentent chacun l'hydrogène ou un halogène,

$R^3$ représente l'hydrogène, un halogène ou un groupe nitro,

$R^6$ et $R^7$, ayant des significations identiques ou différentes, représentent chacun l'hydrogène, un halogène ou un groupe alkyle en $C_1$–$C_6$,

$R^8$ et $R^9$, ayant des significations identiques ou différentes, représentent chacun l'hydrogène ou un halogène, et

$R^{10}$ représente un groupe chlorodifluorométhylthio, trifluorométhylthio, chlorodifluorométhoxy ou trifluorométhoxy, avec les conditions supplémentaires suivantes:

(1) lorsque X représente l'oxygène, et

$R^2$, $R^3$, $R^4$, $R^6$, $R^8$ et $R^9$ l'hydrogène, et

$R^7$ l'hydrogène ou un halogène, et que

(a) $R^1$ et $R^5$ représentent tous des halogènes, ou bien

(b) $R^1$ représente un halogène, un groupe nitro

ou alkyle en $C_1$–$C_6$ et $R^5$ l'hydrogène, dans de tels cas,

$R^{10}$ représente toujours un groupe chlorodifluorométhylthio ou trifluorométhylthio, et

(2) lorsque X représente l'oxygène ou le soufre, et

$R^6$ le chlore, et

$R^8$ l'hydrogène ou le chlore, et

$R^9$ l'hydrogène, et

$R^{10}$ un groupe chlorodifluorométhylthio, trifluoromethylthio, chlorodifluorométhoxy ou trifluorométhoxy, et que

(a) $R^1$ et $R^3$ représentent des halogènes, et $R^2$, $R^4$ et $R^5$ l'hydrogène, ou bien

(b) $R^5$ et $R^3$ représentent des halogènes et $R^1$, $R^2$ et $R^4$ l'hydrogène, ou bien

(c) $R^1$ et $R^4$ représentent des halogènes et $R^2$, $R^3$ et $R^5$ l'hydrogène, ou bien

(d) $R^2$ et $R^5$ représentent des halogènes et $R^1$, $R^3$ et $R^4$ l'hydrogène, dans de tels cas,

$R^7$ représente toujours l'hydrogène ou un groupe alkyle en $C_1$–$C_6$, caractérisé en ce que:

a) on fait réagir des anilines substituées de formule II

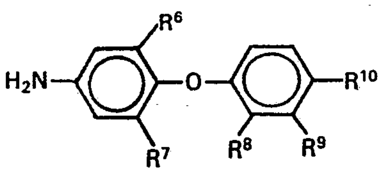

(II)

dans laquelle

$R^6$, $R^7$, $R^8$, $R^9$ et $R^{10}$ ont les significations indiquées ci-dessus, avec des benzoyl-iso(thio)cyanates de formule III

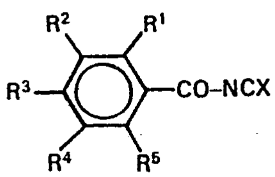

(III)

dans laquelle

X, $R^1$, $R^2$, $R^3$, $R^4$ et $R^5$ ont les significations indiquées ci-dessus, éventuellement en présence d'un diluant, ou bien

b) on fait réagir des phényl-iso(thio)cyanates substitués de formule IV

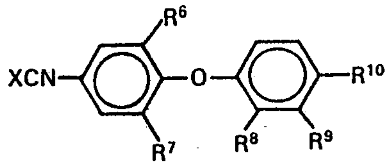

(IV)

dans laquelle

X, $R^6$, $R^7$, $R^8$, $R^9$ et $R^{10}$ ont les significations indiquées ci-dessus, avec des benzamides de formule V

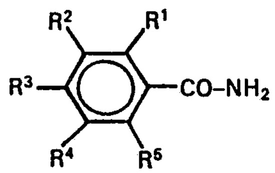

(V)

dans laquelle

$R^1$, $R^2$, $R^3$, $R^4$ et $R^5$ ont les significations indiquées ci-dessus, éventuellement en présence d'un catalyseur et éventuellement en présence d'un diluant.

6. Produit pesticide, caractérisé en ce qu'il contient au moins un composé de formule I selon les revendications 1 ou 5.

7. Utilisation des composés de formule I selon les revendications 1 ou 5 dans la lutte contre les parasites, en particulier les insectes et les arachnides.

8. Procédé pour combattre les parasites, caractérisé en ce que l'on fait agir des composés de formule I selon les revendications 1 ou 5 sur les parasites, de préférence des insectes ou des arachnides, ou leur habitat.

9. Procédé de préparation de produits pesticides, caractérisé en ce que l'on mélange des composés de formule I selon les revendications 1 ou 5 avec des diluants et/ou des agents tensioactifs.

## Claims

1. 1-Phenyl-3-benzoyl-(thio)ureas of the formula (I)

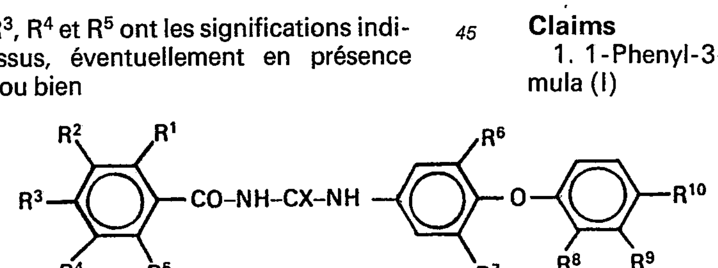

(I)

in which

X represents sulphur or oxygen,

$R^1$ represents hydrogen, halogen, nitro or a radical from the series comprising $C_1$–$C_6$-alkyl and $C_1$–$C_6$-alkylthio,

$R^2$, $R^4$ and $R^5$ are identical or different and represent hydrogen or halogen,

$R^3$ represents hydrogen, halogen or nitro,

$R^6$ and $R^7$ are identical or different and represent hydrogen, halogen or a $C_1$–$C_6$-alkyl radical,

$R^8$ and $R^9$ are identical or different and represent hydrogen or halogen and

$R^{10}$ represents chlorodifluoromethylthio, trifluoromethylthio, chlorodifluoromethoxy or trifluoromethoxy, and

(1) if X represents oxygen and

$R^2$, $R^3$, $R^4$, $R^6$, $R^8$ and $R^9$ represent hydrogen and

$R^7$ represents hydrogen or halogen and

(a) $R^1$ and $R^5$ both represent halogen or

(b) $R^1$ represents halogen, nitro or $C_1$–$C_6$-alkyl and

$R^5$ represents hydrogen, in these cases the radical

$R^{10}$ always denotes chlorodifluoromethylthio or trifluoromethylthio, and

(2) if X represents oxygen or sulphur and

$R^6$ represents chlorine and

$R^8$ represents hydrogen or chlorine and

$R^9$ represents hydrogen and

$R^{10}$ represents chlorodifluoromethylthio, trifluoromethylthio, chlorodifluoromethoxy or trifluoromethoxy and

(a) $R^1$ and $R^3$ represent halogen and $R^2$, $R^4$ and $R^5$ represent hydrogen or

(b) $R^5$ and $R^3$ represent halogen and $R^1$, $R^2$ and $R^4$ represent hydrogen or

(c) $R^1$ and $R^4$ represent halogen and $R^2$, $R^3$ and $R^5$ represent hydrogen or

(d) $R^2$ and $R^5$ represent halogen and $R^1$, $R^3$ and $R^4$ represent hydrogen, in these cases the radical

$R^7$ always denotes hydrogen or $C_1$–$C_6$-alkyl.

2. Compounds of the formula (I) according to Claim 1, in which

X represents oxygen or sulphur,

$R^1$ represents hydrogen, fluorine, chlorine, bromine, nitro, methyl, ethyl, n-propyl, i-propyl, n-butyl, i-butyl, sec.-butyl, tert.-butyl, methylthio, ethylthio, n-propylthio, i-propylthio, n-butylthio, i-butylthio, sec.-butylthio or tert.-butylthio,

$R^2$, $R^4$ and $R^5$ are identical or different and represent hydrogen, fluorine, chlorine or bromine,

$R^3$ represents hydrogen, fluorine, chlorine, bromine or nitro,

$R^6$ and $R^7$ are identical or different and represent hydrogen, fluorine, chlorine, bromine, methyl, ethyl, n-propyl, i-propyl, n-butyl, i-butyl, sec.-butyl or tert.-butyl,

$R^8$ and $R^9$ are identical or different and represent hydrogen, fluorine, chlorine or bromine and

$R^{10}$ represents trifluoromethylthio or trifluoromethoxy, and

(1) if X represents oxygen and

$R^2$, $R^3$, $R^4$, $R^6$, $R^8$ and $R^9$ represent hydrogen and

$R^7$ represents hydrogen, fluorine, chlorine or bromine and

(a) $R^1$ and $R^5$ both represent fluorine, chlorine and/or bromine or

(b) $R^1$ represents fluorine, chlorine, bromine, nitro or the abovementioned alkyl radicals and $R^5$ represents hydrogen, in these cases the radical

$R^{10}$ always denotes trifluoromethylthio, and

(2) if X represents oxygen or sulphur and

$R^6$ represents chlorine and

$R^8$ represents hydrogen or chlorine and

$R^9$ represents hydrogen and

$R^{10}$ represents trifluoromethylthio or trifluoromethoxy and

(a) $R^1$ and $R^3$ represent fluorine, chlorine or bromine and $R^2$, $R^4$ and $R^5$ represent hydrogen or

(b) $R^5$ and $R^3$ represent fluorine, chlorine or bromine and $R^1$, $R^2$ and $R^4$ represent hydrogen or

(c) $R^1$ and $R^4$ represent fluorine, chlorine or bromine and $R^2$, $R^3$ and $R^5$ represent hydrogen or

(d) $R^2$ and $R^5$ represent fluorine, chlorine or bromine and $R^1$, $R^3$ and $R^4$ represent hydrogen, in these cases the radical

$R^7$ always denotes hydrogen or the abovementioned alkyl radicals.

3. Compounds according to Claims 1 and 2, in which X of the formula I according to Claim 1 represents oxygen.

4. Compounds of the formula (I) according to Claim 1, in which

X represents oxygen,

$R^1$ represents hydrogen, fluorine, chlorine, bromine, nitro, methyl or methylthio,

$R^2$, $R^4$ and $R^5$ represent hydrogen, fluorine, chlorine or bromine,

$R^3$ represents hydrogen, fluorine, chlorine or nitro,

$R^6$ and $R^7$ are identical or different and represent hydrogen, chlorine or methyl,

$R^8$ and $R^9$ are identical or different and represent hydrogen, fluorine or chlorine and

$R^{10}$ represents trifluoromethylthio or trifluoromethoxy, and

(1) if $R^2$, $R^3$, $R^4$, $R^6$, $R^8$ and $R^9$ represent hydrogen and

$R^7$ represents hydrogen or chlorine and

(a) $R^1$ and $R^5$ both represent fluorine, chlorine and/or bromine or

(b) $R^1$ represents fluorine, chlorine, bromine, nitro or methyl and $R^5$ represents hydrogen, in these cases the radical

$R^{10}$ always denotes trifluoromethylthio, and

(2) if $R^6$ represents chlorine and

$R^8$ represents hydrogen or chlorine and

$R^9$ represents hydrogen and

$R^{10}$ represents trifluoromethylthio or trifluoromethoxy and

(a) $R^1$ and $R^3$ represent fluorine, chlorine and/or bromine and $R^2$, $R^4$ and $R^5$ represent hydrogen or

(b) $R^5$ and $R^3$ represent fluorine, chlorine and/or bromine and $R^1$, $R^2$ and $R^4$ represent hydrogen or

(c) $R^1$ and $R^4$ represent fluorine, chlorine and/or bromine and $R^2$, $R^3$ and $R^5$ represent hydrogen and

(d) $R^2$ and $R^5$ represent fluorine, chlorine and/or bromine and $R^1$, $R^3$ and $R^4$ represent hydrogen, in these cases the radical $R^7$ always denotes hydrogen or methyl.

5. Process for the preparation of 1-phenyl-3-benzoyl(thio)ureas of the formula (I)

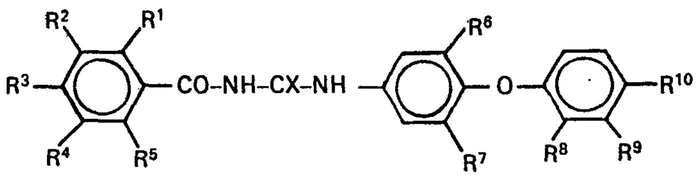

(I)

in which

X represents sulphur or oxygen,

$R^1$ represents hydrogen, halogen, nitro or a radical from the series comprising $C_1$–$C_6$-alkyl and $C_1$–$C_6$-alkylthio,

$R^2$, $R^4$ and $R^5$ are identical or different and represent hydrogen or halogen,

$R^3$ represents hydrogen, halogen or nitro,

$R^6$ and $R^7$ are identical or different and represent hydrogen, halogen or a $C_1$–$C_6$-alkyl radical,

$R^8$ and $R^9$ are identical or different and represent hydrogen or halogen and

$R^{10}$ represents chlorodifluoromethylthio, trifluoromethylthio, chlorodifluoromethoxy or trifluoromethoxy, and

(1) if X represents oxygen and

$R^2$, $R^3$, $R^4$, $R^6$, $R^8$ and $R^9$ represent hydrogen and

$R^7$ represents hydrogen or halogen and

(a) $R^1$ and $R^5$ both represent halogen or

(b) $R^1$ represents halogen, nitro or $C_1$–$C_6$-alkyl and

$R^5$ represents hydrogen, in these cases the radical

$R^{10}$ always denotes chlorodifluoromethylthio or trifluoromethylthio, and

(2) if X represents oxygen or sulphur and

$R^6$ represents chlorine and

$R^8$ represents hydrogen or chlorine and

$R^9$ represents hydrogen and

$R^{10}$ represents chlorodifluoromethylthio, trifluoromethylthio, chlorodifluoromethoxy or trifluoromethoxy, and

(a) $R^1$ and $R^3$ represent halogen and $R^2$, $R^4$ and $R^5$ represent hydrogen or

(b) $R^5$ and $R^3$ represent halogen and $R^1$, $R^2$ and $R^4$ represent hydrogen or

(c) $R^1$ and $R^4$ represent halogen and $R^2$, $R^3$ and $R^5$ represent hydrogen or

(d) $R^2$ and $R^5$ represent halogen and $R^1$, $R^3$ and $R^4$ represent hydrogen, in these cases the radical

$R^7$ always denotes hydrogen or $C_1$–$C_6$-alkyl, characterised in that

a) substituted anilines of the formula (II)

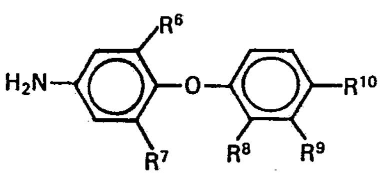

(II)

in which

$R^6$, $R^7$, $R^8$, $R^9$ and $R^{10}$ have the meanings given above, are reacted with benzoyl-iso(thio)cyanates of the formula (III)

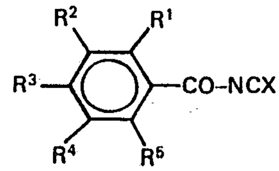

(III)

in which

X, $R^1$, $R^2$, $R^3$, $R^4$ and $R^5$ have the meanings given above, if appropriate in the presence of a diluent, or

b) substituted phenyl-iso(thio)cyanates of the formula (IV)

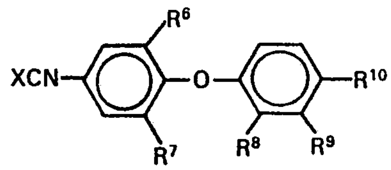

(IV)

in which

X, $R^6$, $R^7$, $R^8$, $R^9$ and $R^{10}$ have the meanings given above, are reacted with benzoic acid amides of the formula (V)

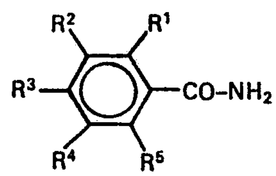

(V)

in which

$R^1$, $R^2$, $R^3$,,$R^4$ and $R^5$ have the meaning given above, if appropriate in the presence of a catalyst and, if appropriate, in the presence of a diluent.

6. Pest-combating agents, characterised in that they contain at least one compound of the formula I according to Claims 1 or 5.

7. Use of compounds of the formula I according to Claims 1 or 5 for combating pests, in particular insects and arachnida.

8. Process for combating pests, characterised in that compounds of the formula I according to Claims 1 or 5 are allowed to act on the pests, preferably insects or arachnida, or on their habitat.

9. Process for the preparation of pest-combating agents, characterised in that compounds of the formula I according to Claims 1 or 5 are mixed with extenders and/or surface-active agents.